(19) 

Europäisches Patentamt
European Patent Office
Office européen des brevets

(11) **EP 3 985 099 A1**

(12) **EUROPEAN PATENT APPLICATION**
published in accordance with Art. 153(4) EPC

(43) Date of publication:
**20.04.2022 Bulletin 2022/16**

(21) Application number: **20840744.5**

(22) Date of filing: **10.07.2020**

(51) International Patent Classification (IPC):
*C12M 1/42* (2006.01)    *C12P 1/00* (2006.01)
*C12N 5/071* (2010.01)    *C12N 15/87* (2006.01)

(52) Cooperative Patent Classification (CPC):
**C12M 1/42; C12N 15/87; C12P 1/00**

(86) International application number:
**PCT/JP2020/027109**

(87) International publication number:
**WO 2021/010341 (21.01.2021 Gazette 2021/03)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **12.07.2019   JP 2019130712**

(71) Applicant: FUJIFILM Corporation
**Tokyo 106-8620 (JP)**

(72) Inventors:
• **NAGAI, Yoichi**
  **Ashigarakami-gun, Kanagawa 258-8577 (JP)**
• **TAKAHASHI, Naoto**
  **Ashigarakami-gun, Kanagawa 258-8577 (JP)**
• **EDO, Michiko**
  **Ashigarakami-gun, Kanagawa 258-8577 (JP)**

(74) Representative: **Hoffmann Eitle**
**Patent- und Rechtsanwälte PartmbB**
**Arabellastraße 30**
**81925 München (DE)**

(54) **PRODUCTION METHOD OF ORGANISM-DERIVED SUBSTANCE, PRODUCTION METHOD OF PRODUCT, AND VOLTAGE APPLICATION DEVICE**

(57)    The producing method according to the disclosed technology is a producing method for an organism-derived material into which a bioactive substance has been introduced, the producing method comprising a step of causing a suspension containing the organism-derived material before the introduction of the bioactive substance and containing the bioactive substance to pass through a first electric field region having a first electric field intensity; and a step of causing the suspension to pass through a second electric field region having a second electric field intensity lower than the first electric field intensity after the suspension has passed through the first electric field region. The organism-derived material is a human-derived cell.

FIG. 2

EP 3 985 099 A1

**Description**

**BACKGROUND OF THE INVENTION**

1. Field of the Invention

The disclosed technology relates to a producing method for an organism-derived material, a producing method for a product, and a voltage applying device.

2. Description of the Related Art

The electroporation method is a method of introducing a substance into a cell by making pores in a cell membrane with an electric pulse. For example, in a case where an electric pulse is applied to a cell suspension to make micropores in a cell membrane, and deoxyribonucleic acid (DNA) is introduced into a cell, it is possible to transform the cell. The following techniques are known as the technique for introducing a bioactive substance such as DNA into a cell using the electroporation method.
For example, JP2004-500022A discloses a device in which electrodes are arranged in a flow channel through which cells flow. JP2015-8708A describes that in a method of introducing a foreign gene into a eukaryotic algae cell by the electroporation method, rectangle-shaped wave electric pulses having three conditions different from each other are stepwisely applied to a solution containing green algae cells and a nucleic acid molecule.

**SUMMARY OF THE INVENTION**

A batch process is widely used as a process of introducing a bioactive substance such as DNA, ribonucleic acid (RNA), or a protein into an organism-derived material such as a cell, a cell derivative, a cell organelle, an intracellular granule, or a vesicle using the electroporation method. In the batch process, for example, a suspension containing an organism-derived material and a bioactive substance is accommodated inside a vessel in which a pair of parallel plate electrodes is provided on the inner wall, and a predetermined voltage is applied to the parallel plate electrodes. As a result, micropores are opened in the membrane covering the surface of the organism-derived material, and thus the permeability of the membrane is increased. In addition, here, in a case where a bioactive substance passes through the membrane of which the permeability has been increased, by diffusion or electrophoresis, the bioactive substance is introduced into the organism-derived material.
In the electroporation method, it is known that in a case where the inter-electrode distance between the parallel plate electrodes becomes excessive, the introduction efficiency of the bioactive substance decreases. In the batch process, the reaction space inside the vessel is limited by the inter-electrode distance, and thus the treatable amount of suspension decreases in a case where the inter-electrode distance between the parallel plate electrodes is shortened. That is, it is difficult to improve the treatment efficiency in the batch process, and it is conceived that the batch process is not suitable for treating a large amount of suspension.
On the other hand, a flow process, in which a suspension containing an organism-derived material and a bioactive substance is caused to flow in a flow channel in which parallel plate electrodes are arranged, is conceived to be suitable for treating a large amount of suspension. In the flow process, a suspension passes through an electric field region that is formed in the flow channel by applying a voltage to the parallel plate electrodes. However, in the flow process, since the suspension flows inside the flow channel, the diffusion or the electrophoresis for introducing a bioactive substance into the organism-derived material is inhibited as compared with the batch process. As a result, in the flow process, the introduction efficiency of the bioactive substances decreases as compared with the batch process.
An object of one aspect of the disclosed technology is to increase the introduction efficiency of a bioactive substance into an organism-derived material.
The producing method for a organism-derived material according to the disclosed technology is a producing method for an organism-derived material into which a bioactive substance has been introduced, the producing method comprising a step of causing a suspension containing the organism-derived material before the introduction of the bioactive substance and containing the bioactive substance to pass through a first electric field region having a first electric field intensity; and a step of causing the suspension to pass through a second electric field region having a second electric field intensity lower than the first electric field intensity after the suspension has passed through the first electric field region. The organism-derived material may be a human-derived cell and is most preferably a HEK293 cell. According to the producing method according to the disclosed technology, it is possible to increase the introduction efficiency of a bioactive substance into an organism-derived material. In the present specification, the introduction efficiency [%] is determined according to the following; the number of cells into which a bioactive substance has been introduced $\times$ 100/(the number of live cells + the number of dead cells). "The number of live cells + the number of dead cells" indicates the total number of cells used in the analysis.

A first period during which a suspension passes through a first electric field region is preferably equal to a second period during which the suspension passes through a second electric field region or shorter than the second period, and a ratio T1/T2 of the first period T1 to the second period T2 is more preferably 1/1,000 or more and 1 or less. This makes it possible to promote the effect of increasing the introduction efficiency of a bioactive substance into an organism-derived material. In a case where the voltage is applied as direct current, T1 is a period obtained by dividing L1 by the average flow speed calculated from the average flow rate and the cross section area. In a case where the voltage is applied as pulses, it is the total value of the pulse times of the pulses applied during the period obtained by dividing L1 by the average flow speed calculated from the average flow rate and the cross section area. In addition, in a case where the voltage is applied as direct current, T2 is similarly a period obtained by dividing L2 by the average flow speed calculated from the average flow rate and the cross section area. In a case where the voltage is applied as pulses, it is the total value of the pulse times of the pulses applied during the period obtained by dividing L2 by the average flow speed calculated from the average flow rate and the cross section area.

In addition, the ratio E2/E1 of the second electric field intensity E2 to the first electric field intensity E1 is preferably 1/1,000 or more and less than 1, more preferably 1/10 or more and less than 1, and particularly preferably 1/10 or more and less than 1/4. This makes it possible to promote the effect of increasing the introduction efficiency of a bioactive substance into an organism-derived material and makes it possible to increase the cell survival rate. The electric field intensity can be determined by dividing the voltage value measured by an oscilloscope by the inter-electrode distance.

A suspension containing a bioactive substance and an organism-derived material may pass through an electric field-free region after passing through the first electric field region and before passing through the second electric field region. This makes it possible to reduce the stress on the organism-derived material as compared with a case where the suspension passes through the second electric field region immediately after passing through the first electric field region.

The ratio T1/T0 of the first period T1 during which a suspension passes through the first electric field region to the third period T0 during which the suspension passes through the electric field-free region is preferably 1/25,000 or more and less than 1, and still more preferably 1/25,000 or more and 1/10 or less. This makes it possible to promote the effect of reducing stress on the organism-derived material. In a case where T1/T0 is set to 1/10 or less, it is possible to suppress the decrease in cell viability due to the accumulation of damage to cells due to heat generation. T0 is preferably 50 ms or more and 5 minutes or less, and more preferably 2 minutes. In a case where T0 is set to 50 ms or more, it is possible to prevent excessive heat generation and improve the cell survival rate. In a case where T0 is set to 5 minutes or less, it is possible to improve the introduction efficiency by causing cells to pass through the second electric field region before the cell pores generated by causing the cells to pass through the first electric field region are closed. T0 is determined by dividing L0 by the average flow speed calculated from the average flow rate and the cross section area.

A suspension containing the bioactive substance and a suspension containing the organism-derived material before the introduction of the bioactive substance flow through flow channels different from each other may be mixed at a merging poit of the respective flow channels and then may pass through the first electric field region and the second electric field region. In a case where the two kinds of suspensions are allowed to flow through separate flow channels and then combined, it is possible to promote the mixing of the organism-derived material with the bioactive substance.

The suspension passes through the first electric field region and the second electric field region by flowing inside the flow channel, and in a case where an area of a cross section of the flow channel orthogonal to a flow direction of the suspension is denoted by S [m$^2$], a circumference length of the cross section of the flow channel is denoted by C [m], and an average speed at which the suspension passes through the first electric field region and the second electric field region is denoted by u [m/s], a shear rate D [s$^{-1}$] defined by Expression (1) is preferably 1 [s$^{-1}$] or more and 5,000 [s$^{-1}$] or less, more preferably 1 [s$^{-1}$] or more and 2,000 [s$^{-1}$] or less, and most preferably 1 [s$^{-1}$] or more and 1,000 [s$^{-1}$] or less. Within this range, it is possible to increase the viability of cells that are vulnerable to shearing.

$$D = 2u \times C/S \cdots (1)$$

In a case where the shear rate D is set to the above range, it is possible to suppress the shear stress that acts on the organism-derived material and increase the flow amount of the suspension per time, when the organism-derived material contained in the suspension flows through the flow channel.

A step of causing the suspension to pass through at least one electric field region different from the first electric field region and the second electric field region may be further provided. This makes it possible to more finely set the conditions of the electric field in the electric field region through which the suspension passes and makes it possible to further increase the introduction efficiency of a bioactive substance into an organism-derived material.

The bioactive substance may be DNA.

The producing method for a product according to the disclosed technology includes a step of culturing the organism-derived material produced by the above-described producing method; and a step of extracting a product that is produced by the organism-derived material. According to the producing method according to the disclosed technology, it is possible to increase the introduction efficiency of a bioactive substance into an organism-derived material, and thus it is possible to increase the production efficiency of a product. The product that is produced by an organism-derived material that is a human-derived cell may be a virus.

The voltage applying device according to the disclosed technology includes a flow channel for causing a liquid to flow; a first pair of electrodes provided to face each other on wall surfaces of the flow channel; and a second pair of electrodes provided to face each other on the wall surfaces of the flow channel, downstream of the first pair of electrodes in a flow direction of the liquid. The length of the first pair of electrodes in the flow direction is equal to or shorter than the length of the second pair of electrodes in the flow direction. According to the voltage applying device according to the disclosed technology, it is possible to increase the introduction efficiency of a bioactive substance into an organism-derived material. The length of the first pair of electrodes may be 0.1 cm or more and 30 cm or less, preferably 0.2 cm or more and 10 cm or less, and more preferably 0.5 cm or more and 5 cm or less. The length of the second pair of electrodes may be 0.1 cm or more and 30 cm or less, preferably 0.2 cm or more and 10 cm or less, and more preferably 0.5 cm or more and 5 cm or less.

The ratio L1/L2 of the length L1 of the first pair of electrodes in the flow direction to the length L2 of the second pair of electrodes in the flow direction is preferably 1/1,000 or more and 1 or less, preferably 1/200 or more and 1/2 or less, and most preferably 1/100 or more and 1/10 or less. This makes it possible to promote the effect of increasing the introduction efficiency of a bioactive substance into an organism-derived material. It is good for the first pulse to be short; however, in a case where L1/L2 is set to 1/1,000 or more, the electrode processing becomes easy.

The ratio L1/L0 of the length L1 of the first pair of electrodes in the flow direction to the length L0 between the first pair of electrodes and the second pair of electrodes in the flow direction is preferably 1/30,000 or more and 1/10 or less, and most preferably 1/25,000 or more and 1/100 or less. This makes it possible to promote the effect of reducing stress on the organism-derived material.

Each of the inter-electrode distance of the first pair of electrodes and the inter-electrode distance of the second pair of electrodes is preferably 10 $\mu$m or more and less than 10 mm, more preferably 20 $\mu$m or more and 7 mm or less, and most preferably 1 mm or more and 5 mm or less. This makes it possible to prevent the voltage that is applied to the first pair of electrodes and the second pair of electrodes from becoming excessively high and to prevent the cross section area of the flow channel from becoming excessively small. In a case of 1 mm or more, the flow channel is not clogged even with the concentrated cell solution, and it is possible to decrease the voltage in a case of 5 mm or less.

It is preferable that a first voltage is applied to the first pair of electrodes and a second voltage lower than the first voltage is applied to the second pair of electrodes. This makes it possible to increase the introduction efficiency of a bioactive substance into an organism-derived material.

The voltage applying device according to the disclosed technology may further include at least one pair of electrodes provided to face each other on the wall surfaces of the flow channel, which is different from the first pair of electrodes and the second pair of electrodes. This makes it possible to more finely set the conditions of the electric field in the electric field region through which the suspension passes and makes it possible to further increase the introduction efficiency of a bioactive substance into an organism-derived material. Depending on the size and the electrical properties of the bioactive substance to be introduced, there is present an electric field suitable for the introduction. For this reason, an electric field region may be provided according to the number of genes to be introduced. For example, in a case where three or more genes are introduced, the electric field region may be two or may be three.

The flow channel may have at least one merging poit or a branch point. This makes it possible to promote the mixing of the organism-derived material with the bioactive substance in a case where the two kinds of suspensions are allowed to flow through separate flow channels and then combined.

According to the disclosed technology, it is possible to increase the introduction efficiency of a bioactive substance into an organism-derived material.

**BRIEF DESCRIPTION OF THE DRAWINGS**

Fig. 1A is a plan view illustrating an example of a configuration of a voltage applying device according to an embodiment of the disclosed technology.
Fig. 1B is a cross-sectional view taken along a line 1B-1B in Fig. 1A.
Fig. 1C is a cross-sectional view taken along a line 1C-1C in Fig. 1A.
Fig. 2 is an enlarged view illustrating a part of Fig. 1B.
Fig. 3 is a plan view illustrating an example of a configuration of a voltage applying device according to another embodiment of the disclosed technology.

Fig. 4 is a plan view illustrating an example of a configuration of a voltage applying device according to another embodiment of the disclosed technology.

Fig. 5A is a plan view illustrating an example of a configuration of a voltage applying device according to another embodiment of the disclosed technology.

Fig. 5B is a cross-sectional view taken along a line 5B-5B in Fig. 5A.

Fig. 6 is an enlarged view illustrating a part of Fig. 5B.

Fig. 7A is a diagram illustrating an example of the relative relationship of electric field intensity in each electric field region according to an embodiment of the disclosed technology.

Fig. 7B is a diagram illustrating an example of the relative relationship of electric field intensity in each electric field region according to an embodiment of the disclosed technology.

Fig. 7C is a diagram illustrating an example of the relative relationship of electric field intensity in each electric field region according to an embodiment of the disclosed technology.

Fig. 7D is a diagram illustrating an example of the relative relationship of electric field intensity in each electric field region according to an embodiment of the disclosed technology.

Fig. 8 is a cross-sectional view illustrating an example of a configuration of a voltage applying device 1C according to a modified example.

Fig. 9 is a view illustrating an example of a configuration of a product producing device according to another embodiment of the disclosed technology.

Fig. 10 is a graph showing the gene introduction efficiency and the cell viability acquired from each sample.

## DESCRIPTION OF THE PREFERRED EMBODIMENTS

Hereinafter, embodiments of the present disclosed technology will be described with reference to the drawings. In each of the drawings, substantially the same or equivalent configuration elements or parts are designated by the same reference numeral. In the present specification, the organism-derived material is a human-derived cell. The cells may be a human T cell, HEK293, A549, SF9, EB66, Daudi, Hela, Vero, or MDCK. The bioactive substance is a substance such as a DNA, an RNA, or a protein, where the bioactive substance exhibits some action on an organism-derived material by being introduced into the organism-derived material. Examples thereof include a plasmid, a linear DNA, an mRNA, and a protein, and a plasmid, an mRNA, or a linear DNA is particularly preferable.

[First embodiment]

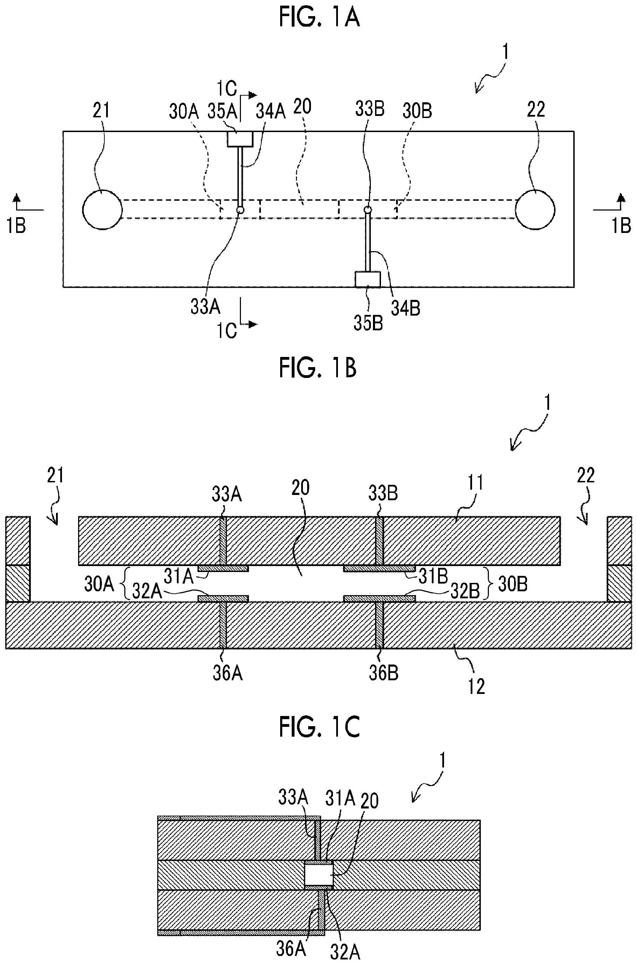

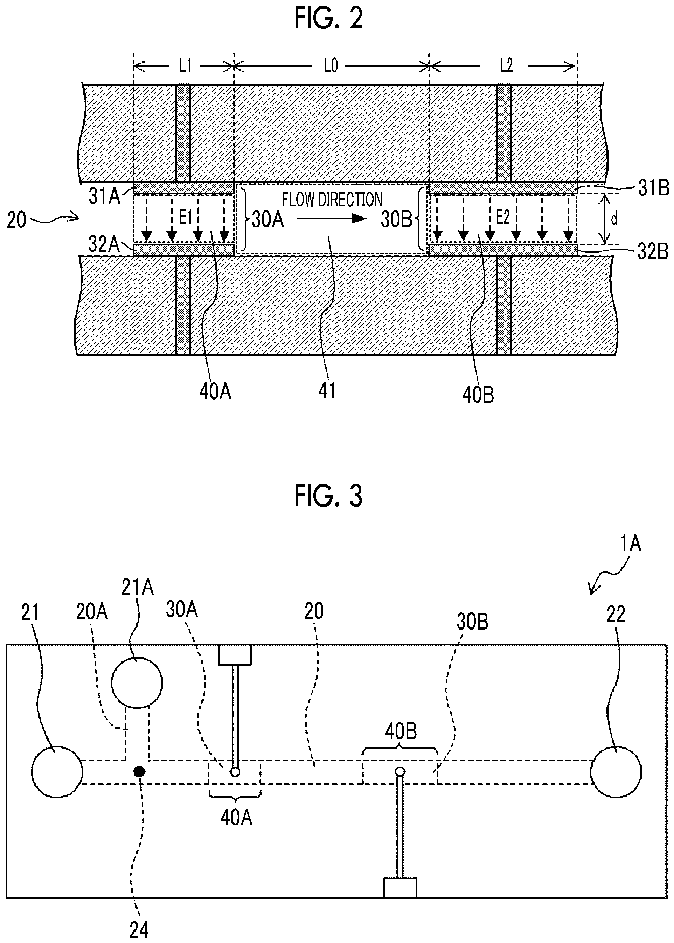

Fig. 1A is a plan view illustrating an example of a configuration of a voltage applying device 1 according to a first embodiment of the disclosed technology. Fig. 1B is a cross-sectional view taken along a line 1B-1B in Fig. 1A. Fig. 1C is a cross-sectional view taken along a line 1C-1C in Fig. 1A. Fig. 2 is an enlarged view illustrating a part of Fig. 1B.

The voltage applying device 1 is a device that is used for introducing a bioactive substance into an organism-derived material by the electroporation method. It becomes possible to prepare a novel cell having novel genetic characteristics, for example, by introducing DNA, which is an example of the bioactive substance, into a cell, which is an example of the organism-derived material.

As illustrated in Fig. 1B, the voltage applying device 1 has an upper wall part 11 and a lower wall part 12, which are provided to face each other. The voltage applying device 1 has a flow channel 20 formed between the upper wall part 11 and the lower wall part 12. An inflow port 21 and an outflow port 22, which communicate with the flow channel 20, are provided in the upper wall part 11. The inflow port 21 is provided on one end side of the flow channel 20, and the outflow port 22 is provided on the other end side of the flow channel 20. When the voltage applying device 1 is used, a suspension (hereinafter, simply referred to as a suspension) containing an organism-derived material before the introduction of a bioactive substance and containing a bioactive substance is injected into the inflow port 21, flows through the flow channel 20, and then flows out of the outflow port 22. In the present embodiment, the area of the cross section of the flow channel 20 orthogonal to the flow direction of the suspension is set to be constant, and thus the flow speed of the suspension that flows through the flow channel 20 is set to be constant.

An upper electrode 31A is provided on the surface of the upper wall part 11 on the flow channel 20 side, and a lower electrode 32A facing the upper electrode 31A is provided on the surface of the lower wall part 12 on the flow channel 20 side. The upper electrode 31A and the lower electrode 32A form a pair of electrodes 30A. The pair of electrodes 30A has the form of a so-called parallel plate electrode. The pair of electrodes 30A is an example of the first pair of electrodes in the disclosed technology.

A via 33A consisting of a conductor that is connected to the upper electrode 31A and penetrates in the thickness direction of the upper wall part 11 is provided in the upper wall part 11. A wire 34A and a pad 35A, which are electrically connected to the upper electrode 31A through the via 33A, are provided on the surface of the upper wall part 11 opposite to the flow channel 20. Similarly, a via 36A consisting of a conductor that is connected to the lower electrode 32A and penetrates in the thickness direction of the lower wall part 12 is provided in the lower wall part 12. A wire and a pad (not illustrated in the drawing), which are electrically connected to the lower electrode 32A through the via 36A, are provided on the surface of the lower wall part 12 opposite to the flow channel 20. In a case where a voltage is applied to this pad and the pad 35A provided in the upper wall part 11 by using an external power supply, a voltage is applied to the pair of electrodes 30A, and then an electric field region 40A having an electric field intensity E1 is formed in the space between the upper electrode 31A and the lower electrode 32A (see Fig. 2). The electric field region 40A is an example of the first electric field region in the disclosed technology. The direction of the electric field in the electric field region 40A may be, for example, a direction from the upper electrode 31A toward the lower electrode 32A.

In addition, on the surface of the upper wall part 11 on the flow channel 20 side, the upper electrode 31B is provided downstream of the pair of electrodes 30A in the flow direction (hereinafter, simply referred to as the flow direction) of the suspension that flows through the flow channel 20. The lower electrode 32B facing the upper electrode 31B is provided on the surface of the lower wall part 12 on the flow channel 20 side. The upper electrode 31B and the lower electrode 32B form a pair of electrodes 30B. The pair of electrodes 30B has the form of a so-called parallel plate electrode. The pair of electrodes 30B is an example of the second pair of electrodes in the disclosed technology.

A via 33B consisting of a conductor that is connected to the upper electrode 31B and penetrates in the thickness direction of the upper wall part 11 is provided in the upper wall part 11. A wire 34B and a pad 35B, which are electrically connected to the upper electrode 31B through the via 33B, are provided on the surface of the upper wall part 11 opposite to the flow channel 20. Similarly, a via 36B consisting of a conductor that is connected to the lower electrode 32B and penetrates in the thickness direction of the lower wall part 12 is provided in the lower wall part 12. A wire and a pad (not illustrated in the drawing), which are electrically connected to the lower electrode 32B through the via 36B, are provided on the surface of the lower wall part 12 opposite to the flow channel 20. In a case where a voltage is applied to this pad and the pad 35B provided in the upper wall part 11 by using an external power supply, a voltage is applied to the pair of electrodes 30B, and then an electric field region 40B having an electric field intensity E2 is formed in the space between the upper electrode 31B and the lower electrode 32B (see Fig. 2). The electric field region 40B is an example of the second electric field region in the disclosed technology. The direction of the electric field in the electric field region 40B may be, for example, a direction from the upper electrode 31B toward the lower electrode 32B.

A voltage VI is applied to the pair of electrodes 30A, and a voltage V2 lower than the voltage VI is applied to the pair of electrodes 30B. The voltage that is applied to the pair of electrodes 30A and the pair of electrodes 30B may be a direct current voltage or an alternating current voltage. In the present embodiment, the inter-electrode distances d of the pair of electrodes 30A and the pair of electrodes 30B are set to be the same, and thus the electric field intensity E2 of the electric field region 40B formed by the pair of electrodes 30B is set to be lower than the electric field intensity E1 of the electric field region 40A formed by the pair of electrodes 30A. As a result, the electrophoresis of the bioactive substance is promoted while the membrane permeability of the organism-derived material is maintained in the electric field region 40B. The electric field intensity E1 is an example of the first electric field intensity in the disclosed technology, and the electric field intensity E2 is an example of the second electric field intensity in the disclosed technology.

Here, in a case where the inter-electrode distances d of the pair of electrodes 30A and the pair of electrodes 30B become excessively long, the voltage that is applied to the pair of electrodes 30A and the pair of electrodes 30B becomes high in order to obtain the desired electric field intensity in the electric field region 40A and the electric field region 40B. In a case where the voltage that is applied to the pair of electrodes 30A and the pair of electrodes 30B becomes excessively high, the pair of electrodes 30A and the pair of electrodes 30B deteriorate easily. On the other hand, in a case where the inter-electrode distances d of the pair of electrodes 30A and the pair of electrodes 30B become excessively short, the cross section area of the flow channel 20 becomes small, and thus it becomes difficult to treat a large amount of suspension. The inter-electrode distances d of the pair of electrodes 30A and the pair of electrodes 30B are preferably 10 $\mu$m or more and less than 10 mm. This makes it possible to prevent the voltage that is applied to the pair of electrodes 30A and the pair of electrodes 30B from becoming excessively high and to prevent the cross section area of the flow channel 20 from becoming excessively small.

The suspension that flows through the flow channel 20 passes through the electric field region 40A and then passes through the electric field region 40B. The length L1 of the pair of electrodes 30A in the flow direction is preferably equal to or shorter than the length L2 of the pair of electrodes 30B in the flow direction (L1 $\leq$ L2). In other words, the period T1 during which a suspension passes through the electric field region 40A is preferably equal to or shorter than the period T2 during which the suspension passes through the electric field region 40B. In addition, the ratio L1/L2 of the length L1 of the pair of electrodes 30A in the flow direction to the length L2 of the pair of electrodes 30B in the flow direction is preferably 1/1,000 or more and 1 or less. In other words, the ratio T1/T2 of the period T1 during which a suspension passes through the electric field region 40A to the period T2 during which the suspension passes through the electric field region 40B is preferably 1/1,000 or more and 1 or less. The period T1 is an example of the first period in the disclosed technology, and the period T2 is an example of the second period in the disclosed technology.

Further, in the region between the pair of electrodes 30A and the pair of electrodes 30B, no pair of electrodes is provided, and an electric field-free region 41 in which the electric field intensity is substantially zero is formed. Here, the description that the electric field intensity is substantially zero means that the influence of the electric field in the electric field region 40A and the electric field region 40B, which are adjacent to the electric field-free region 41, is capable of reaching the electric field-free region 41. The length L1 of the pair of electrodes 30A in the flow direction is preferably shorter than the length L0 of the region between the pair of electrodes 30A and the pair of electrodes 30B in the flow direction (that is, the electric field-free region 41) (L1 <L0). In other words, the period T1 during which a suspension passes through the electric field region 40A is preferably equal to or shorter than the period T0 during which the suspension passes through the electric field-free region 41. In addition, the ratio L1/L0 of the length L1 of the pair of electrodes 30A in the flow direction to the length L0 of the region between the pair of electrodes 30A and the pair of electrodes 30B in the flow direction is preferably 1/1,000 or more and less than 1. In other words, the ratio T1/T0 of the period T1 during which a suspension passes through the electric field region 40A to the period T0 during which the suspension passes through the electric field-free region 41 is preferably 1/1,000 or more and less than 1. Within the above range, it is possible to relax the influence of the electric field region on another different electric field region.

In addition, in a case where an area of a cross section of the flow channel 20 orthogonal to a flow direction of the suspension is denoted by S [m$^2$], a circumference length of the cross section of the flow channel 20 is denoted by C [m], and an average speed at which the suspension passes through the electric field region 40A and the electric field region 40B is denoted by u [m/s], a shear rate D [s$^{-1}$] defined by Expression (1) is preferably 1 [s$^{-1}$] or more and 5,000 [s$^{-1}$] or less, more preferably 1 [s$^{-1}$] or more and 2,000 [s$^{-1}$] or less, and still preferably 1 [s$^{-1}$] or more and 1,000 [s$^{-1}$] or less. In a case where the shear rate D is set to the above range, it is possible to suppress the shear stress that acts on the organism-derived material and ensure the flow amount of the suspension per time, when the organism-derived material contained in the suspension flows through the flow channel 20.

$$D = 2u \times C/S \cdots (1)$$

When the voltage applying device 1 is used, a voltage VI is applied to the pair of electrodes 30A, and a voltage V2 lower than the voltage VI is applied to the pair of electrodes 30B. Then, the suspension containing the organism-derived material before the introduction of the bioactive substance and containing the bioactive substance is injected into the inflow port 21. The suspension injected into the inflow port 21 flows out of the outflow port 22 through the flow channel 20. By using the voltage applying device 1, the producing method for an organism-derived material according to the embodiment of the disclosed technology described below is realized.

The producing method for an organism-derived material according to the embodiment of the disclosed technology include a step of causing a suspension containing the organism-derived material before the introduction of the bioactive substance and the bioactive substance to pass through an electric field region 40A having an electric field intensity E1; and a step of causing the suspension to pass through an electric field region 40B having an electric field intensity E2 lower than the electric field intensity E1 after the suspension has passed through the electric field region 40A. While the suspension injected from the inflow port 21 flows through the flow channel 20, the treatment in each of the above steps are carried out. That is, according to the voltage applying device 1, the introduction of the bioactive substance into the organism-derived material by the electroporation method is carried out by the flow process.

In a case where the suspension passes through the electric field region 40A having a relatively high electric field intensity, pores are opened in the membrane (for example, the cell membrane) that covers the surface of the organism-derived material, and thus the permeability of the membrane increases. At this time, in a case where a part of bioactive substances pass through the membrane of which the permeability has been increased, by diffusion or electrophoresis, the bioactive substance is introduced into the organism-derived material. Then, the suspension passes through the electric field region 40B having a relatively low electric field intensity, whereby the electrophoresis of the bioactive substance further occurs while membrane permeability is maintained, and the introduction of the bioactive substance into the organism-derived material is promoted. That is, according to the voltage applying device 1 and the producing method for an organism-derived material according to the embodiment of the disclosed technology, it is possible to increase the introduction efficiency of the bioactive substance into the organism-derived material as compared with the case where the suspension passes through a single electric field region.

The total energy E [J/μL] per unit volume that is applied to the suspension while the suspension passes through both the electric field regions 40A and 40B is applied according to Expression (2). In Expression (2), VI [V] is the voltage value of the voltage pulse that is applied to the pair of electrodes 30A, I1 [A] is the current value that flows through the pair of electrodes 30A in a case where the voltage pulse is applied to the pair of electrodes 30A, and T1 [sec] is the pulse width of the voltage pulse that is applied to the pair of electrodes 30A. V2 [V] is the voltage value of the voltage pulse that is applied to the pair of electrodes 30B, 12 [A] is the current value that flows through the pair of electrodes 30B in a case where the voltage pulse is applied to the pair of electrodes 30B, T2 [sec] is the pulse width of the voltage pulse that is applied to the pair of electrodes 30B, and M [μL] is the volume of the suspension that passes through the electric field regions 40A and 40B.

$$E = (V1 \times I1 \text{ x } T1 + V2 \times I2 \times T2)/M \cdots (2)$$

The total energy E that is applied to the suspension has a strong influence on the introduction efficiency of the bioactive substance into the organism-derived material and the survival rate of the organism-derived material. For increasing the introduction efficiency of the bioactive substance and the survival rate of the organism-derived material, the total energy E that is applied to the suspension is preferably 0.01 [J/μL] or more and 0.30 [J/μL] or less, more preferably 0.01 [J/μL] or more and 0.20 [J/μL] or less, and most preferably 0.02 [J/μL] or more and 0.13 [J/μL] or less.

In addition, in a case where length L1 of the pair of electrodes 30A in the flow direction is set to be equal to or shorter than the length L2 of the pair of electrodes 30B in the flow direction, it is possible to make the period T1 during which a suspension passes through the electric field region 40A having a high electric field be equal to or shorter than the period T2 during which the suspension passes through the electric field region 40B having a low electric field. In a case where the period T2 is set to be equal to or longer than the period T1, it is possible to promote the introduction of the bioactive substance into the organism-derived material by electrophoresis. In a case where L1/L2 or T1/T2 is set to be 1/1,000 or more and 1 or less, the effect of increasing the introduction efficiency of the bioactive substance into the organism-derived material is remarkably exhibited. For example, in a case where a voltage pulse having a relatively long pulse width is required, as the voltage pulse that is applied to the pair of electrodes 30B, in order to form the electric field region 40B having a low electric field, the following is preferably satisfied; L1 ≤ L2 or T1 ≤ T2. On the other hand, in a case where a voltage pulse that is applied to the pair of electrodes 30A is required to be applied over a plurality of times in order to form the electric field region 40A having a high electric field, the following is preferably satisfied; L1 > L2 or T1 > T2.

In addition, according to the voltage applying device 1 and the producing method for an organism-derived material according to the embodiment of the disclosed technology, the suspension passes through the electric field-free region 41 after passing through the electric field region 40A and before passing through the electric field region 40B. In a case where the suspension passes through the electric field region 40B via the electric field-free region 41, after passing through the electric field region 40A having a relatively high electric field intensity, it is possible to suppress the influence of the electric field region 40A on the electric field region 40B.

In addition, according to the voltage applying device 1 according to the embodiment of the disclosed technology, in a case where each of the ratio L1/L0 of the length L1 of the pair of electrodes 30A in the flow direction to the length L0 between the pair of electrodes 30A and the pair of electrodes 30B in the flow direction and the ratio T1/T0 of the period T1 during which a suspension passes through the electric field region 40A to the period T0 during which the suspension passes through the electric field-free region 41 set to 1/1,000 or more and less than 1, the effect of each electric field region is remarkably exhibited.

[Second embodiment]

Fig. 3 is a plan view illustrating an example of a configuration of a voltage applying device 1A according to a second embodiment of the disclosed technology.

The voltage applying device 1A has two inflow ports 21 and 21A. The inflow port 21A communicates with the flow channel 20A, and the flow channel 20A is connected to the flow channel 20. That is, the flow channel 20 has a merging poit 24 where the flow channel 20 is combined with the flow channel 20A.

When the voltage applying device 1A is used, for example, the suspension containing the organism-derived material before the introduction of the bioactive substance is injected into the inflow port 21, and the suspension containing the bioactive substance is injected into the inflow port 21A. Two kinds of suspensions flow through the flow channels 20A and 20 different from each other, are mixed at the merging poit 24, and then pass through the electric field region 40A and the electric field region 40B. In a case where the two kinds of suspensions are allowed to flow through separate flow channels and then to be mixed in this manner, it is possible to suppress the damage to the organism-derived material and increase the survival rate of the organism-derived material. Furthermore, since this leads to the improvement of the survival rate of the introduced cells, it is possible to increase the introduction efficiency of the bioactive substance into the organism-derived material.

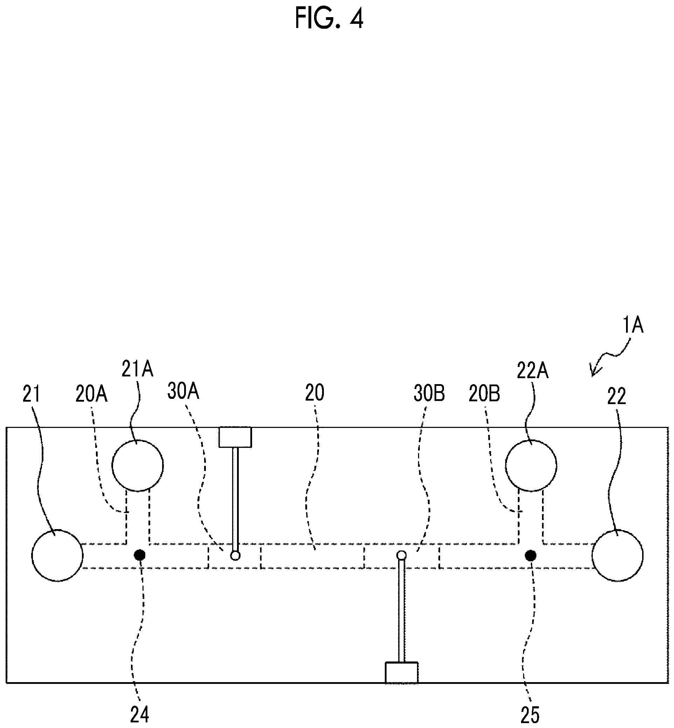

In addition, as illustrated in Fig. 4, the voltage applying device 1A may have two outflow ports 22 and 22A. The outflow port 22A communicates with the flow channel 20B, and the flow channel 20B is connected to the flow channel 20. That is, the flow channel 20 has a branch point 25 that branches to the flow channel 20B. According to the voltage applying device 1A illustrated in Fig. 4, the suspension that has passed through the electric field region 40A and the electric field region 40B flows out of each of the outflow ports 22 and 22A. In a case where two outflow ports are provided as described above, it is possible to supply to a plurality of culture containers.

[Third embodiment]

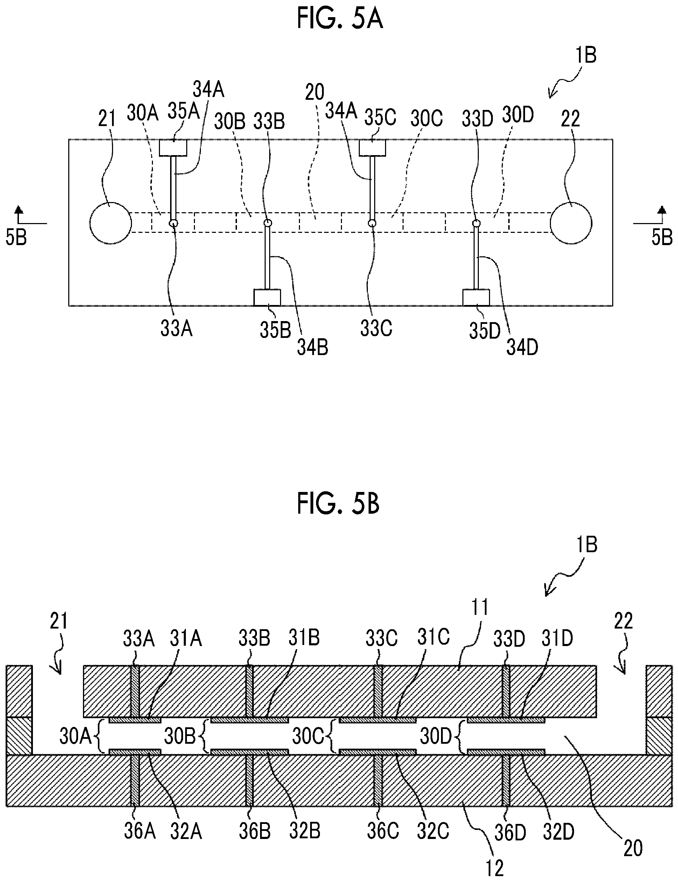

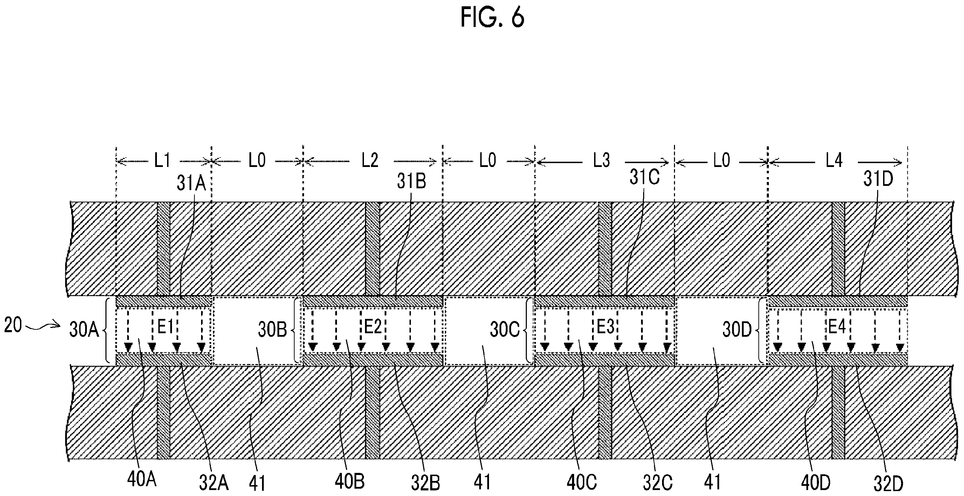

Fig. 5A is a plan view illustrating an example of a configuration of a voltage applying device 1B according to a third embodiment of the disclosed technology. Fig. 5B is a cross-sectional view taken along a line 5B-5B in Fig. 5A. The voltage applying device 1B further includes a pair of electrodes 30C and a pair of electrodes 30D in addition to the pair of electrodes 30A and the pair of electrodes 30B. In the present embodiment, pairs of electrodes 30A, 30B, 30C, and 30D are provided in order from the upstream side in the flow direction of the suspension. Fig. 6 is an enlarged view illustrating a part of Fig. 5B.

The pair of electrodes 30C and the pair of electrodes 30D respectively have the same forms of the parallel plate electrode as the pair of electrodes 30A and the pair of electrodes 30B. That is, the pair of electrodes 30C has a configuration including an upper electrode 31C provided on the surface of the upper wall part 11 on the flow channel 20 side, and a lower electrode 32C facing the upper electrode 31C and provided on the surface of the lower wall part 12 on the flow channel 20 side. Similarly, the pair of electrodes 30D has a configuration including an upper electrode 31D provided on the surface of the upper wall part 11 on the flow channel 20 side, and a lower electrode 32D facing the upper electrode 31D and provided on the surface of the lower wall part 12 on the flow channel 20 side.

A wire 34C and a pad 35C, which are electrically connected to the upper electrode 31C through the via 33C, are provided on the surface of the upper wall part 11 opposite to the flow channel 20. A wire and a pad (not illustrated in the drawing), which are electrically connected to the lower electrode 32C through the via 36C, are provided on the surface of the lower wall part 12 opposite to the flow channel 20. In a case where a voltage is applied to this pad and the pad 35C provided in the upper wall part 11 by using an external power supply, a voltage is applied to the pair of electrodes 30C, and then an electric field region 40C having an electric field intensity E3 is formed in the space between the upper electrode 31C and the lower electrode 32C (see Fig. 6).

Similarly, a wire 34D a pad 35D, which are electrically connected to the upper electrode 31D through the via 33D, are provided on the surface of the upper wall part 11 opposite to the flow channel 20. A wire and a pad (not illustrated in the drawing), which are electrically connected to the lower electrode 32D through the via 36D, are provided on the surface of the lower wall part 12 opposite to the flow channel 20. In a case where a voltage is applied to this pad and the pad 35D provided in the upper wall part 11 by using an external power supply, a voltage is applied to the pair of electrodes 30D, and then an electric field region 40D having an electric field intensity E4 is formed in the space between the upper electrode 31D and the lower electrode 32D (see Fig. 6). The suspension that flows through the flow channel 20 passes through the electric field regions 40A, 40B, 40C, and 40D in this order.

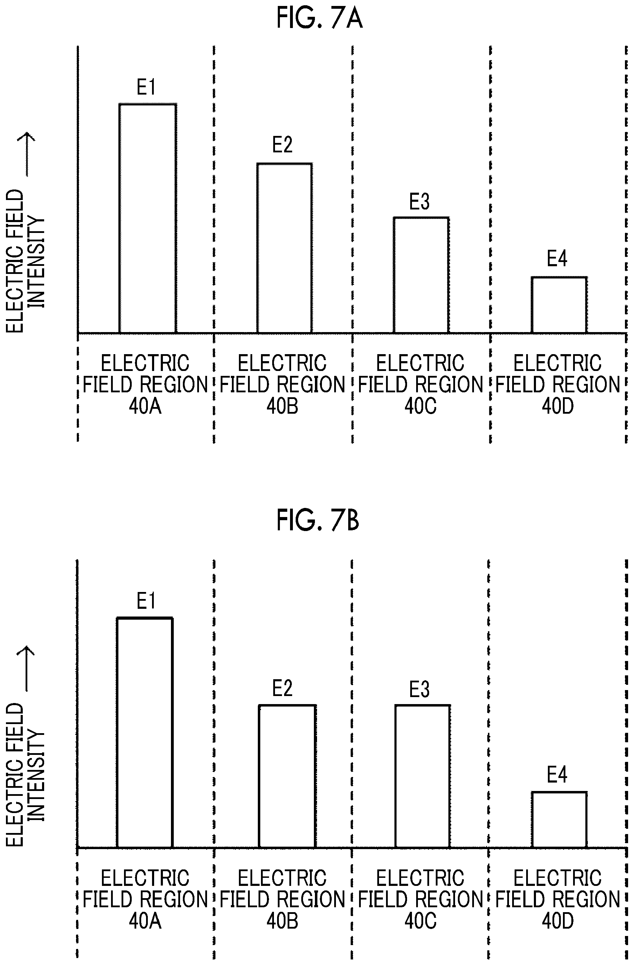

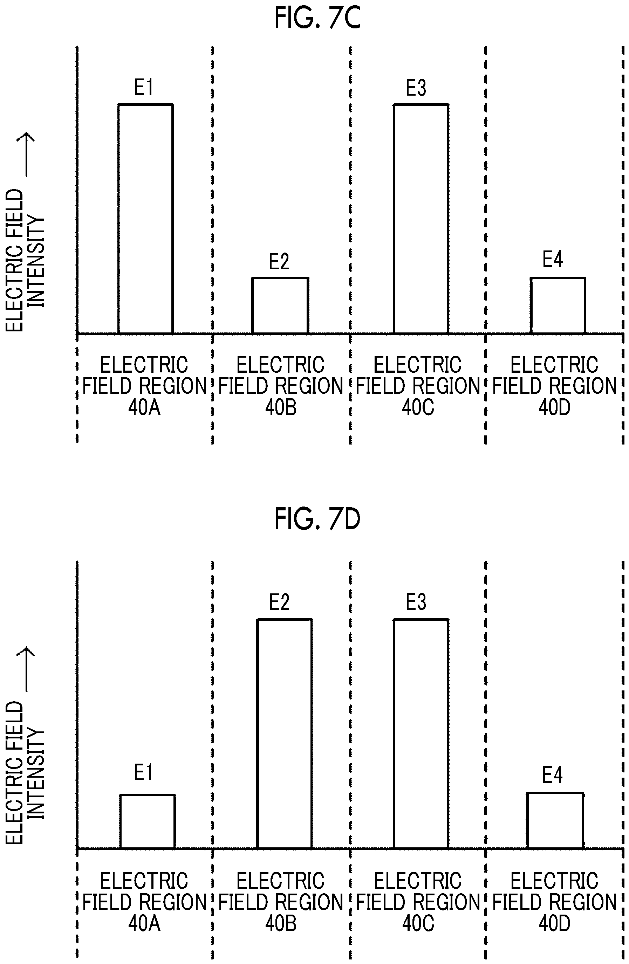

Fig. 7A to 7D are diagrams each illustrating an example of the relative relationship between the electric field intensities E1 to E4 in the electric field regions 40A to 40D. For example, as illustrated in Fig. 7A and Fig. 7B, the electric field intensities E1 to E4 may be set so that the electric field intensity stepwisely decreases from the upstream side to the downstream side in the flow direction of the suspension. In the example illustrated in Fig. 7A, the relationship of E1 > E2 > E3 > E4 is established for the electric field intensities E1 to E4. In the example illustrated in Fig. 7B, the relationship of E1 > E2 = E3 > E4 is established. In addition, as illustrated in Fig. 7C, the electric field intensities E1 to E4 may be set so that regions having a relatively high electric field intensity and regions having a relatively low electric field intensity are alternately lined up along the flow direction of the suspension. In the example illustrated in Fig. 7C, the relationship of E1 = E3 > E2 = E4 is established for the electric field intensities E1 to E4. In addition, as illustrated in Fig. 7D, the electric field intensities E1 to E4 may be set so that the electric field intensity stepwisely decreases from the upstream side to the downstream side in the flow direction of the suspension after the electric field intensity has stepwisely increased. In the example illustrated in Fig. 7D, the relationship of E1 < E2 = E3 > E4 is established for the electric field intensities E1 to E4. In the voltage applying device 1B according to the present embodiment, it is sufficient that the electric field intensities E1 to E4 are set so that a section in which the electric field region having a relatively low electric field intensity is arranged, in the flow direction, adjacent to the downstream side of the electric field region having a relatively high electric field intensity is present at least at one place in the flow channel 20.

The lengths L1 to L4 of the pair of electrodes 30A to 30D in the flow direction may be configured to become short as the electric field intensity formed in the pair of electrodes increases. In other words, the periods T1 to T4 during which the suspension passes through electric field regions 40A to 40D, respectively, may be configured to become short as the electric field intensity in the electric field region increases. For example, as illustrated in Fig. 7A, in a case where the relationship of E1 > E2 > E3 > E4 is established for the electric field intensities E1 to E4, the lengths L1 to L4 of the pairs of electrodes 30A to 30D in the flow direction may be set so that $L1 \leq L2 \leq L3 \leq L4$ is satisfied. Alternatively, the periods T1 to T4 during which the suspension passes through the electric field regions 40A to 40D, respectively, may be set so that $T1 \leq T2 \leq T3 \leq T4$ is satisfied.

In addition, in a case where the length of the pair of electrodes that forms the electric field region in which the electric field intensity is maximized in the flow direction is denoted by $L_{min}$, and the length of the pair of electrodes that forms the electric field region in which the electric field intensity in the flow direction is minimized is denoted by $L_{max}$, it is preferable that the ratio $L_{min}/L_{max}$ of $L_{min}$ to $L_{max}$ is 1/1,000 or more and 1 or less. In other words, the period during which the suspension passes through the electric field region in which the electric field intensity is maximized is denoted by $T_{min}$, and the period during which the suspension passes through the electric field region in which the electric field intensity is minimized is denoted by $T_{max}$, it is preferable that the ratio $T_{min}/T_{max}$ of $T_{min}$ to $T_{max}$ is 1/1,000 or more and 1 or less. This makes it possible to suppress the membrane pore opening to the extent of irreparable opening due to the maximum voltage and makes it possible to promote the introduction of the bioactive substance into the organism-derived material by electrophoresis.

In the voltage applying device 1B according to the present embodiment, no pair of electrodes is provided between the pair of electrodes 30A and the pair of electrodes 30B, between the pair of electrodes 30B and the pair of electrodes 30C, and between the pair of electrodes 30C and the pair of electrodes 30D, and the electric field-free region 41 in which the electric field intensity is substantially zero is formed. In the present embodiment, the lengths L0 of the respective regions (that is, the respective electric field-free regions 41) between the pairs of electrodes adjacent to each other are set to equal to each other.

The length $L_{min}$ of the pair of electrodes that forms the electric field region in which the electric field intensity in the flow direction is maximized is preferably shorter than the length L0 of the electric field-free region 41 in the flow direction. In other words, the period $T_{min}$ during which the suspension passes through the electric field region in which the electric field intensity is maximized is preferably equal to or shorter than the period T0 during which the suspension passes through the electric field-free region 41. In addition, it is preferable that each of the ratio $L_{min}/L0$ of $L_{min}$ to L0 and the ratio of $T_{min}/T0$ of $T_{min}$ to T0 is 1/1,000 or more and less than 1. Within the above range, it is possible to relax the influence of the electric field region on another different electric field region.

In the producing method for an organism-derived material according to the present embodiment, realized by using the voltage applying device 1B, the suspension containing the organism-derived material before the introduction of the bioactive substance and containing the bioactive substance passes through three or more electric field regions. This makes it possible to more finely set the conditions of the electric field in the electric field region through which the suspension passes and makes it possible to further increase the introduction efficiency of a bioactive substance into an organism-derived material.

In the present embodiment, the case where the suspension passes through four electric field regions is exemplified; however, the electric field regions through which the suspension passes may be three or five or more regions.

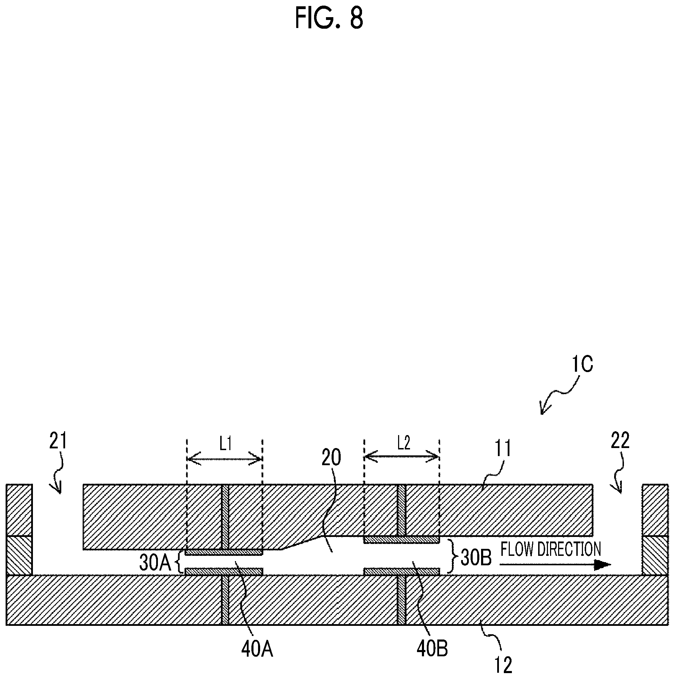

Further, in the first to third embodiments described above, the case where the period during which the suspension passes through each of the electric field regions is adjusted by the length of each of the pairs of electrodes in the flow direction is exemplified; however, this aspect does not limit the above embodiments. Fig. 8 is a cross-sectional view illustrating an example of a configuration of a voltage applying device 1C according to a modified example. As illustrated in Fig. 8, the cross section area of the flow channel 20 in the electric field region 40A having a relatively high electric field intensity may be smaller than the cross section area of the flow channel 20 in the electric field region 40B having a relatively low electric field intensity. As a result, the speed at which the suspension passes through the electric field region 40A is faster than the speed at which the suspension passes through the electric field region 40B. For this reason, for example, in a case where the length L1 of the pair of electrodes 30A in the flow direction and the length L2 of the pair of electrodes 30B in the flow direction are the same, the period T1 during which the suspension passes through the electric field region 40A is shorter than the period T2 during which the suspension passes through the electric field region 40B. In this way, it is possible to adjust the period during which the suspension passes through each of the electric field regions with the cross section area of the flow channel 20 as well.

Further, in the first to third embodiments, the case where the introduction of the bioactive substance into the organism-derived material by the electroporation method is carried out by the flow process is exemplified; however, the introduction may be carried out by the batch treatment. In the batch treatment, for example, in a case where a step in which a suspension containing an organism-derived material and a bioactive substance passes through one electric field region is denoted by one treatment unit, this step is carried out over a plurality of times by stepwisely changing the electric field intensity.

[Fourth embodiment]

Hereinafter, a producing method for a product according to a fourth embodiment of the disclosed technology will be described. The producing method for a product according to the present embodiment includes a step of culturing the organism-derived material (that is the organism-derived material into which the bioactive substance has been introduced) produced by the producing method according to the first to third embodiments described above; and a step of extracting a product that is produced by the cultured organism-derived material. The product may be a vector of virus such as adenovirus, adeno-associated virus, lentivirus, retrovirus, vaccinia virus, herpesvirus, human papillomavirus, and Sendai virus. An adeno-associated virus is most preferable.

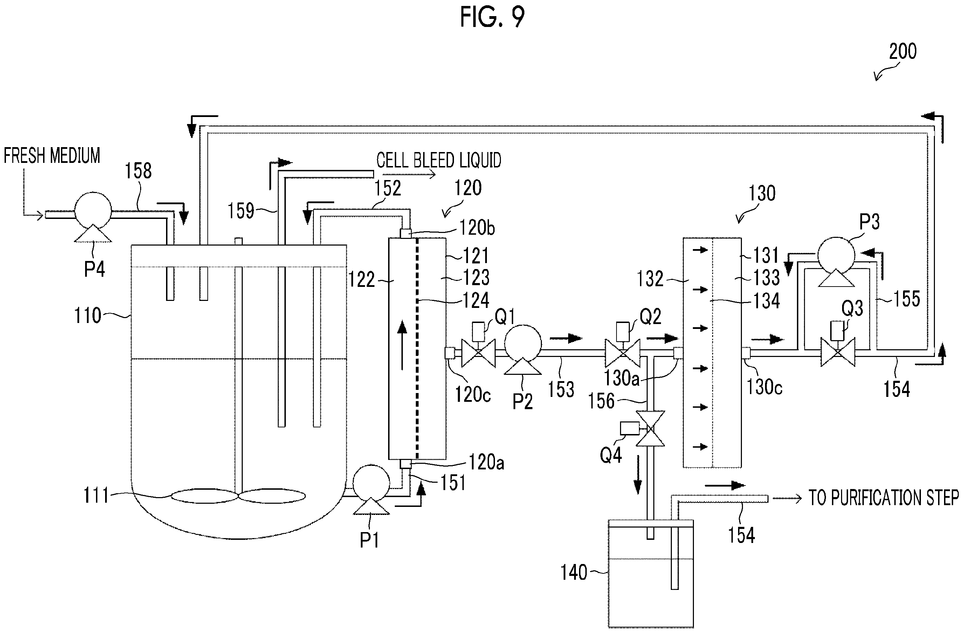

Fig. 9 is a view illustrating an example of a configuration of a product producing device 200 according to a fourth embodiment of the disclosed technology. In the following description, a case where an antibody is produced using an antibody-producing cell with a product producing device 200 will be exemplified. That is, the antibody-producing cell is an organism-derived material into which a DNA useful for producing an antibody having a desired quality has been introduced by the producing method according to the first to third embodiments described above.

The cell that is used for expressing an antibody is not particularly limited; however, examples thereof include eukaryotic cells such as an animal cell, a plant cell, and yeast, prokaryotic cells such as Bacillus subtilis, and Escherichia coli. An animal cell such as a CHO cell, a BHK-21 cell, or an SP2/0-Ag14 cell is preferable, and a CHO cell is more preferable.

The antibody to be expressed in the animal cell is not particularly limited; however, examples thereof include an anti-IL-6 receptor antibody, an anti-IL-6 antibody, an anti-glypican-3 antibody, an anti-CD3 antibody, an anti-CD20 antibody, an anti-GPIIb/IIIa antibody, an anti-TNF antibody, an anti-CD25 antibody, an anti-EGFR antibody, an anti-Her2/neu antibody, an anti-RSV antibody, an anti-CD33 antibody, an anti-CD52 antibody, an anti-IgE antibody, an anti-CD11a antibody, an anti-VEGF antibody, and an anti-VLA4 antibody. The antibody includes not only monoclonal antibodies derived from animals such as a human, a mouse, a rat, a hamster, a rabbit, and a monkey, but also artificially modified antibodies such as a chimeric antibody, a humanized antibody, and a bispecific antibody.

The obtained antibody or a fragment thereof can be purified homogeneously. The separation and the purification of the antibody or the fragment thereof may be carried out using the separation and the purification method that is used for the ordinary polypeptide. For example, a chromatography column for affinity chromatography or the like, a filter, ultrafiltration, salting out, dialysis, SDS polyacrylamide gel electrophoresis, and isoelectric focusing electrophoresis can be appropriately selected and combined to separate and purify an antibody; however, the present invention is not limited to thereof. The concentration of the obtained antibody can be measured by measuring the absorbance or by an enzyme-linked immunosorbent assay (ELISA).

The product producing device 200 includes a culture container 110 accommodating a cell suspension that contains cells, a first filter unit 120 having a first filter membrane 124 that carries out a membrane separation treatment on the cell suspension extracted from the culture container 110, and a flow channel 152, as a circulation flow channel, through which the components blocked by the first filter membrane 124, are returned to the culture container 110. The product producing device 200 further has a second filter unit 130 having a second filter membrane 134 that carries out a membrane separation treatment on the components of the cell suspension, which have permeated through the first filter membrane 124, and a flow channel 154, as a second circulation flow channel, through which the components that have permeated through the second filter membrane 134, are returned to the culture container 110, and recovery flow channels 156 and 157 through which the components blocked by the second filter membrane 134 are recovered.

The culture container 110 is a container that accommodates a cell suspension containing cells and a medium that is used for the expression of an antibody. A stirring device having a stirring blade 111 is provided in the inside of the culture container 110. In a case where the stirring blade 111 is rotated, the medium accommodated in the culture container 110 together with cells is stirred, and thus the homogeneity of the medium is maintained.

The flow channel 151 has one end connected to the bottom of the culture container 110 and the other end connected to the inflow port 120a of the first filter unit 120. A pump P1 for extracting the cell suspension accommodated in the culture container 110 and sending the extracted cell suspension to the first filter unit 120 is provided in the middle of the flow channel 151.

The first filter unit 120 includes a container 121 and a first filter membrane 124 that divides the space inside the container 121 into a supply side 122 and a permeation side 123 and carries out a membrane separation treatment on the cell suspension extracted from the culture container 110. Further, the first filter unit 120 has, on the supply side 122, an inflow port 120a into which the cell suspension flows and an outflow port 120b from which the cell suspension flows out. The cell suspension extracted from the culture container 110 passes through the first filter membrane 124 while flowing into the inside of the container 121 through the inflow port 120a and flowing out of the outside of the container 121 through the outflow port 120b. The first filter unit 120 carries out the membrane separation treatment by a tangential flow (cross flow) method in which the permeated component is sent to the permeation side while a target liquid for the membrane separation treatment flows along the membrane surface of the first filter membrane 124 (in the direction parallel to the membrane surface). In the tangential flow method, which is a method of membrane separation treatment with the first filter membrane 124, a flow in which the cell suspension extracted from the culture container circulates in one direction in parallel along the membrane surface of the first filter membrane 124 may be formed, or a flow in which the cell suspension alternately reciprocates in parallel along the membrane surface of the first filter membrane 124 may be formed.

The components contained in the cell suspension, which have a relatively large size, do not permeate through the first filter membrane 124, flow out of the outside of the container 121 through the outflow port 120b, and are returned to the inside of the culture container 110 through the flow channel 152. That is, the components of the cell suspension extracted from the culture container 110, which have been blocked by the first filter membrane 124, are returned to the inside of the culture container 110 through the flow channel 152. On the other hand, the relatively small-sized component contained in the cell suspension permeates through the first filter membrane 124 and is discharged to the outside of the container 121 from the discharge port 120c provided on the permeation side 123. A flow channel 153, in which a pump P2 is provided, is connected to the permeation side 123 of the first filter unit 120, and the components discharged to the permeation side 123 are sent to the second filter unit 130 through the flow channel 153.

In the product producing device 200 according to the present embodiment, the first filter membrane 124 is used for the intended purpose of separating cells from components unnecessary for cell culture. Examples of the components unnecessary for cell culture include corpses of dead cells, cell debris, DNA, HCP, antibodies, and waste products. That is, the first filter membrane 124 has separation performance that is suitable for allowing components unnecessary for cell culture such as corpses of dead cells, cell debris, DNA, HCP, antibodies, waste products, and the like to permeate, while blocking the permeation of cells. The size of cells that are cultured in the culture container 110 is assumed to be larger than 20 $\mu$m. In addition, it is assumed that the sizes of the corpses of dead cells and the cell debris are 1 $\mu$m or more and 10 $\mu$m or less. Further, the sizes of DNA, HCP, and the antibody is assumed to be about several tens of nm.

The average pore diameter of the first filter membrane 124 is preferably more than 0 and 20 $\mu$m or less, more preferably 0.05 $\mu$m or more and 10 $\mu$m or less, still more preferably 0.1 $\mu$m or more and 9 $\mu$m or less, and most preferably 2 $\mu$m or more and 8 $\mu$m or less. In a case where the average pore diameter of the first filter membrane 124 is set to 20 $\mu$m or less, it is possible to reduce the risk that cells permeate through the first filter membrane 124, and it is possible to suppress the decrease in the number of cells in the culture container 110. The average pore diameter of the first filter membrane 124 can be measured by a 95% separation particle diameter in a case where a mesh is used or by mercury porosimetry in a case where an MF membrane or a UF membrane is used.

As the first filter membrane 124, it is possible to use a mesh filter formed by weaving a fibrous member in a mesh form. In a case of using a mesh filter as the first filter membrane 124, it is possible to promote the discharge of components unnecessary for cell culture, which includes corpses of dead cells and cell debris, to the permeation side, as compared with a case of using a hollow fiber membrane. As a result, the components unnecessary for cell culture can be effectively removed from the culture container 110, and thus the proliferation of cells in the culture container 110 can be enhanced.

Further, as the first filter membrane 124, a hollow fiber membrane such as a microfiltration membrane or an ultrafiltration membrane can be used. In a case where a hollow fiber membrane is used as the first filter membrane 124, it is possible to reduce the risk that cells permeate to the permeation side as compared with the case of using a mesh filter. Further, it is possible to reduce the risk of the occurrence of clogging caused by the entrance of cells into the first filter membrane 124. This makes it possible to reduce cell loss.

The permeation side of the first filter unit 120 is connected to the supply side 132 of the second filter unit 130 through the flow channel 153. Valves Q1 and Q2 and a pump P2 are provided in the middle of the flow channel 153. The valves Q1 and Q2 are controlled to be in the open state in a case where the permeated solution that has permeated through the first filter membrane 124 is sent from the first filter unit 120 to the second filter unit 130, and they are controlled to be in the close state in cases other than the above.

The second filter unit 130 includes a container 131 and a second filter membrane 134 that divides the space inside the container 131 into a supply side 132 and a permeation side 133 and carries out a membrane separation treatment on the permeated solution that has permeated through the first filter membrane 124. Further, the second filter unit 130 has an inflow port 130a into which the cell suspension flows on the supply side 132. The permeated solution that has permeated through the first filter membrane 124 flows into the inside of the container 131 from the inflow port 130a. In the present embodiment, the second filter unit 130 carries out a membrane separation treatment by a dead-end method in which a substantially entire amount of liquid on the supply side 132 is filtered.

The components contained in the permeated solution passed through the first filter membrane 124, which have a relatively large size, do not permeate through the second filter membrane 134 and remains on the membrane surface of the second filter membrane 134 or on the supply side 132 of the second filter unit 130. On the other hand, the relatively small-sized components contained in the permeated solution that has permeated through the first filter membrane 124 permeate through the second filter membrane 134 and then permeate to the permeation side 133. A discharge port 130c is provided on the permeation side 133 of the second filter unit 130, and a flow channel 154 is connected to the discharge port 130c. The components that have passed through the second filter membrane 134 are discharged from the discharge port 130c to the outside of the container 131 and returned to the culture container 110 through the flow channel 154. One end of the flow channel 154 is connected to the discharge port 130c, and the other end is connected to the culture container 110.

The second filter membrane 134 is used for the intended purpose of separating the medium from components unnecessary for culture including an antibody, which are contained in the permeated solution that has permeated through the first filter membrane 124. That is, the second filter membrane 134 has a separation performance suitable for blocking the permeation of components unnecessary for cell culture including an antibody.

The average pore diameter of the second filter membrane 134 is preferably 1 $\mu$m or less, more preferably 0.1 $\mu$m or less, still more preferably 0.05 $\mu$m or less, and most preferably 0.01 $\mu$m or less. In a case where the average pore diameter of the second filter membrane 134 is set to 1 $\mu$m or less, it is possible to reduce the risk that components unnecessary for cell culture including an antibody return to the culture container 10 through the flow channel 154. The average pore diameter of the second filter membrane 134 can be measured by a 95% separation particle diameter in a case where a mesh is used or by a mercury porosimetry in a case where an MF membrane or a UF membrane is used.

As the second filter membrane 134, a hollow fiber microfiltration membrane (MF membrane) can be used. In a case where a hollow fiber microfiltration membrane is used as the second filter membrane 134, it is possible to reduce the risk of the occurrence of clogging as compared with the case where a hollow fiber ultrafiltration membrane is used.

Further, as the second filter membrane 134, a hollow fiber ultrafiltration membrane (UF membrane) can be used. In a case where a hollow fiber ultrafiltration membrane is used as the second filter membrane 134, it is possible to effectively collect components unnecessary for cell culture including an antibody as compared with the case where a hollow fiber microfiltration membrane is used.

In the middle of the flow channel 154, a valve Q3 is provided in the vicinity of the permeation side 133 of the second filter unit 130. The valve Q3 is controlled to be in the open state in a case where the components that have permeated through the second filter membrane 134 are sent to the culture container 110, and it is controlled to be in the close state in cases other than the above.

The product producing device 200 according to the present embodiment has a recovery unit for recovering components unnecessary for cell culture including an antibody blocked by the second filter membrane 134. The above recovery unit has a configuration including a backwash flow channel 155, a pump P3, recovery flow channels 156 and 157, and a recovery tank 140.

The backwash flow channel 155 forms a bypass flow channel that bypasses the entry side and the exit side of the valve Q3. The pump P3 is provided in the middle of the backwash flow channel 155 and generates a liquid flow directed from the permeation side 133 toward the supply side 132 of the second filter unit 130 in a direction opposite to the liquid flow generated during the normal membrane separation treatment, whereby the second filter membrane 134 is subjected to the backwash treatment. While the backwash treatment is carried out, the valve Q3 is controlled to be in the close state, and the liquid used for the backwash flows through the backwash flow channel 155 and then is supplied to the second filter membrane 134. In a case where the second filter membrane 134 is subjected to the backwash treatment, the components unnecessary for culture including an antibody, which remain on the membrane surface of the second filter membrane 134 and on the supply side 132 of the second filter unit 130, are discharged from the inflow port 130a of the second filter unit 130.

In the vicinity of the inflow port 130a of the second filter unit 130, the recovery flow channel 156 is connected to the flow channel 153 that connects the permeation side 123 of the first filter unit 120 to the supply side 132 of the second filter unit 130. While the backwash treatment is carried out, the valves Q1, Q2, and Q3 are controlled to be in the close state, and the valve Q4 is controlled to be in the open state. As a result, the components unnecessary for culture including an antibody, which are discharged from the inflow port 130a of the second filter unit 130 by the backwash treatment, are accommodated in the recovery tank 140 through the recovery flow channel 156. The components unnecessary for culture including an antibody, which are accommodated in the recovery tank 140, are sent to the antibody purification step, which is the next step, through the recovery flow channel 157.

The product producing device 200 has a medium supply flow channel 158 for supplying a fresh medium to the culture container 110, and a pump P4 provided in the middle of the medium supply flow channel 158. In addition, in the product producing device 200, in order to prevent the concentration of cells in the culture container 110 from becoming excessively high, a cell bleeding treatment in which a part (for example, about 10%) of the cells in the culture container 110 is extracted at an appropriate timing within the culture period is carried out. In the cell bleeding treatment, the cells in the culture container 110 are discharged to the outside of the culture container 110 through the flow channel 159. In addition, the product producing device 200 has a control unit, not illustrated in the drawing, which controls pumps P1 to P4 and valves Q1 to Q4. The operation of the product producing device 200 will be described below.

In a case where the membrane separation treatment is carried out in the first filter unit 120 and the second filter unit 130 in the product producing device 200, the pumps P1 and P2 are made into a driving state, and the pump P3 is made into a stopped state. Further, the valves Q1, Q2, and Q3 are controlled to be in the open state, and the valve Q4 is controlled to be in the close state.

In a case where the pump P1 is made into a driving state, the cell suspension accommodated in the culture container 110 is sent to the supply side 122 of the first filter unit 120. The cell suspension extracted from the culture container 110 is subjected to a membrane separation treatment by the tangential flow method with the first filter membrane 124. The cells blocked by the first filter membrane 124 are returned into the culture container 110 through the flow channel 152. On the other hand, the components unnecessary for culture including an antibody permeate through the first filter membrane 124.

The permeated solution that has permeated through the first filter membrane 124 is sent to the supply side 132 of the second filter unit 130 through the flow channel 153. The permeated solution that has permeated through the first filter membrane 124 is subjected to a membrane separation treatment by the dead-end method with the second filter membrane 134. The components unnecessary for cell culture including an antibody blocked by the second filter membrane 134 remain on the membrane surface of the second filter membrane 134 or on the supply side 132 of the second filter unit 130. On the other hand, the clean medium from which components unnecessary for cell culture such as an antibody have been removed, which have permeated through the second filter membrane 134, is returned to the culture container 110 through the flow channel 154.

On the other hand, in a case where the backwash treatment is carried out in the product producing device 200, the pumps P3 are made into a driving state, and the pumps P1 and P2 are made into a stopped state. Further, the valve Q4 is controlled to be in the open state, and the valves Q1, Q2, and Q3 are controlled to be in the close state.

The driving of the pump P3 generates a liquid flow directed from the permeation side toward the supply side of the second filter unit 130 in a direction opposite to the liquid flow generated during the normal membrane separation treatment, whereby the second filter membrane 134 is subjected to the backwash treatment. In a case where the second filter membrane 134 is subjected to the backwash treatment, the components unnecessary for cell culture including an antibody, which remain on the membrane surface of the second filter membrane 134 and on the supply side 132 of the second filter unit 130, are discharged from the inflow port 130a of the second filter unit 130. The components unnecessary for cell culture including an antibody, which are discharged from the second filter unit 130 by the backwash treatment, are accommodated in the recovery tank 140 through the recovery flow channel 156. The components unnecessary for culture including an antibody, which are accommodated in the recovery tank 140, are sent to the antibody purification step, which is the next step, through the recovery flow channel 157. During the culture period, the pump P3 is driven intermittently, and the backwash treatment is carried out intermittently. As a result, the feeding of liquid of the components unnecessary for cell culture including an antibody to the recovery flow channel 156 is carried out intermittently. In the product producing device 200 according to the present embodiment, the membrane separation treatment and the backwash treatment are alternately and repeatedly carried out during the culture period.

The pump P2 is driven continuously or at a predetermined timing while the membrane separation treatment and the backwash treatment are carried out, and a fresh medium of an amount, which is substantially the same as the amount of the medium sent to the recovery tank 140 through the recovery flow channel 156, is supplied to the culture container 110 through the medium supply flow channel 158. As a result, the amount of the medium in the culture container 110 is kept substantially constant during the culture period.

In a case where the product producing device 200 according to the present embodiment is operated as described above, a producing method for a product, including a step of culturing the organism-derived material (that is the organism-derived material into which the bioactive substance has been introduced) produced by the producing method according to the first to third embodiments described above and a step of extracting a product that is produced by the cultured organism-derived material, is realized.

According to the producing method for a product according to the present embodiment, it is possible to increase the introduction efficiency of a bioactive substance into an organism-derived material, and thus it is possible to increase the production efficiency of a product.

Examples

In indicating the number of cells, "M" shall mean 1,000,000. For example, 1 M means 1,000,000, 0.1 M means 100,000, and 10 M means 10,000,000.

The introduction of a bioactive substance into an organism-derived material was carried out according to the following procedure. First, HEK293 cells (Expi293F cells, Thermo Fisher Scientific, Inc.) were seeded in an Expi293 Expression Medium (Thermo Fisher Scientific, Inc.) so that the cell concentration was 0.5 M cells/mL. This cell suspension was accommodated in an incubator having a $CO_2$ concentration of 8% and an ambient temperature of 37°C and cultured for 1 day with stirring at 120 rpm.

On the next day, after confirming that the cell concentration of the cell suspension was 1 M cells/mL, centrifugation treatment was carried out at 200 × g for 5 minutes. The supernatant of the cell suspension after centrifugation treatment was removed, and the cells were resuspended in a new Expi293 Expression Medium (Thermo Fisher Scientific, Inc.), and the cell concentration was adjusted to 30 M cells/mL. The pmaxGFP (Lonza) was added to this cell suspension so that the concentration thereof was 5 μg/mL, and mixed to obtain a cell-plasmid mixture.

A 50 mL syringe was filled with the cell-plasmid mixture, and 9 mL was subjected to the feeding of liquid to a flow channel having a pair of electrodes at a flow speed of 8 mL/min. A voltage pulse of 130 V and 5 ms was applied to the pair of electrodes within a period of 150 ms, during which the cell-plasmid mixture was retained in the flow channel. That is, a voltage is applied over 5 ms to all the cells that pass through the flow channel, and 145 ms is the rest period. The cell-plasmid mixture that had passed through the flow channel was recovered in a recovery bottle. 6 mL of 9 mL of the cell-plasmid mixture recovered in the recovery bottle was recovered, and the remaining 3 mL was used as a sample 1. The sample 1 is Comparative Example to which the disclosed technology is not applied since the voltage is applied only once.

3 mL of 6 mL of the recovered cell-plasmid mixture was subjected again to the feeding of liquid to the above flow channel at a flow speed of 8 mL/min. A voltage pulse of 20 V and 75 ms was applied to the pair of electrodes within a period of 150 ms, during which the cell-plasmid mixture was retained in the flow channel. That is, a voltage is applied over 75 ms to all the cells that pass through the flow channel, and 75 ms is the rest period. The cell-plasmid mixture that had passed through the flow channel was recovered in a recovery bottle. This recovered cell-plasmid mixture was used as a sample 2. That is, the sample 2 is a sample subjected to the first voltage application with a voltage pulse of 130 V and 5 ms and then the second voltage application with a voltage pulse of 20 V and 75 ms. At this time, the inter-electrode distance at the time of applying the first time pulse and the second time pulse was 1 mm. The conditions are as follows; T1/T2 = 1/15, E2/E1 = 1/6.5, T1/T0 = 1/24,000, shear rate = 400 [s$^{-1}$], and L1/L2 = 1.

The remaining 3 mL of 6 mL of the cell-plasmid mixture, excluding the sample 1, was subjected again to the feeding of liquid to the above flow channel at a flow speed of 8 mL/min. A voltage pulse of 40 V and 75 ms was applied to the pair of electrodes within a period of 150 ms, during which the cell-plasmid mixture was retained in the flow channel. That is, a voltage is applied over 75 ms to all the cells that pass through the flow channel, and 75 ms is the rest period. The cell-plasmid mixture that had passed through the flow channel was recovered in a recovery bottle. This recovered cell-plasmid mixture was used as a sample 3. That is, the sample 3 is a sample subjected to the first voltage application with a voltage pulse of 130 V and 5 ms and then the second voltage application with a voltage pulse of 40 V and 75 ms. The conditions are as follows; T1/T2 = 1/15, E2/E1 = 1/3.25, T1/T0 = 1/24,000, shear rate = 400 [s$^{-1}$], and L1/L2 = 1.

The applied voltage pulses and total energy for the samples 1 to 3 are summarized in Table 1 below. The total energy was calculated using Expression (2). As V1 and T1 in Expression (2), the applied voltage and the pulse width in the first feeding of liquid were applied, respectively. As V2 and T2 in Expression (2), the applied voltage and the pulse width in the second feeding of liquid were applied, respectively. I1 and I2 in the Expression (2) were calculated by dividing the applied voltage by the resistance value (83$\Omega$) between the electrodes. The resistance value between the electrodes was acquired in advance by the following procedure. A 50 mL syringe was filled with a suspension of HEK293 cells (Thermo Fisher Scientific, Inc.), and the cell suspension was subjected to the feeding of liquid to the above flow channel at a flow speed of 8 mL/min, and the above flow channel was filled with the cell suspension. Then, the resistance value between the electrodes provided in the flow channel was measured. The resistance value was 83 $\Omega$. The inter-electrode distance is 1 mm.

[Table 1]

| | First time | | Second time | | Total energy |
|---|---|---|---|---|---|
| | Voltage | Pulse width | Voltage | Pulse width | |
| Sample 1 (Comparative Example) | 130 V | 5 ms | - | - | 0.0509 J/$\mu$L |
| Sample 2 | 130 V | 5 ms | 20 V | 75 ms | 0.0670 J/$\mu$L |
| Sample 3 | 130 V | 5 ms | 40 V | 75 ms | 0.1232 J/$\mu$L |

Samples 1 to 3 recovered in the separate recovery bottles were allowed to stand for 10 minutes and then centrifugation treatment was carried out at 200 × g for 5 minutes. The supernatant of each sample after centrifugation treatment was removed and the cells were resuspended in a new Expi293 medium (Thermo Fisher Scientific, Inc.).

Each sample was added dropwise to a pre-warmed 2 mL of the Expi293 Expression Medium (Thermo Fisher Scientific, Inc.) accommodated in a well plate so that the cell concentration was 1 M cells/mL. Each sample was accommodated in an incubator having a $CO_2$ concentration of 8% and an ambient temperature of 37°C and subjected the stationary culture for 24 hours.

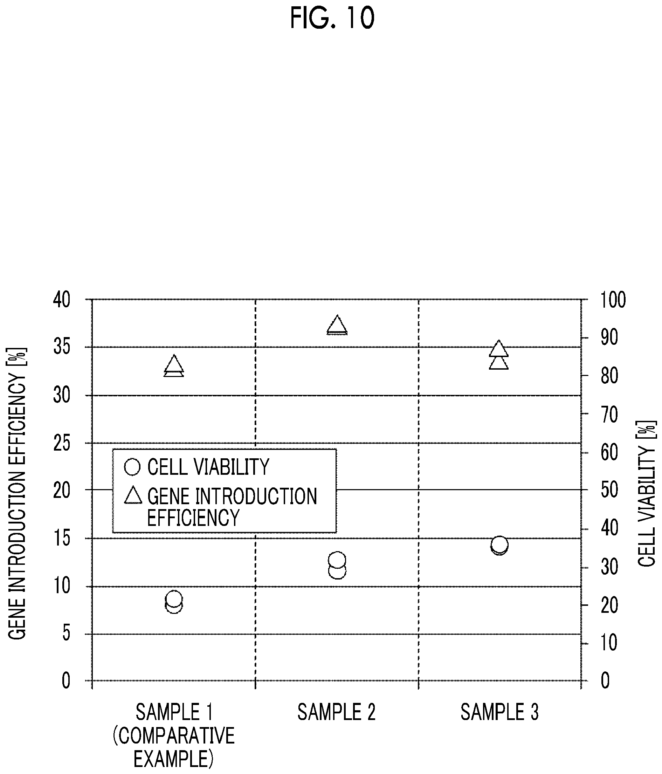

The gene introduction efficiency (transferring rate) of each sample was acquired using BD FACS Calibur (Becton, Dickinson and Company), and the cell viability was acquired using Vi-CELL XR (Beckman Coulter Inc.). Fig. 10 is a graph showing the gene introduction efficiency and the cell viability acquired from the samples 1 to 3. As shown in Fig. 10, in the samples 2 and 3, in which the number of times of application of the voltage pulse is 2, the gene introduction efficiency and the cell viability are improved as compared with the sample 1 in which the number of times of application of the voltage pulse is 1. Furthermore, the sample 2 has high cell survival rate as compared with the sample 3. In addition, the sample 3 has high gene introduction efficiency as compared with the sample 2. Specifically, regarding the introduction efficiency on average, the sample 1 is 8.64%, the sample 2 is 12.36%, and the sample 3 is 14.51%. Regarding the cell viability on average, the sample 1 is 82.03%, the sample 2 is 92.73%, and the sample 3 is 85.97%.

The disclosure of JP2019-130712 filed on July 12, 2019, is incorporated in the present specification by reference in its entirety. In addition, all documents, patent applications, and technical standards described in the present specification are incorporated in the present specification by reference, to the same extent as in the case where each of the documents, patent applications, and technical standards is specifically and individually described.

**Claims**

1. A producing method for an organism-derived material into which a bioactive substance has been introduced, the producing method comprising:

    a step of causing a suspension containing the organism-derived material before the introduction of the bioactive substance and containing the bioactive substance to pass through a first electric field region having a first electric field intensity; and
    a step of causing the suspension to pass through a second electric field region having a second electric field intensity lower than the first electric field intensity after the suspension has passed through the first electric field region,
    wherein the organism-derived material is a human-derived cell.

2. The producing method according to claim 1,
    wherein a first period during which the suspension passes through the first electric field region is equal to or shorter than a second period during which the suspension passes through the second electric field region.

3. The producing method according to claim 2,
    wherein a ratio T1/T2 of the first period T1 to the second period T2 is 1/1,000 or more and 1 or less.

4. The producing method according to any one of claims 1 to 3,
    wherein a ratio E2/E1 of the second electric field intensity E2 to the first electric field intensity E1 is 1/1,000 or more and less than 1.

5. The producing method according to any one of claims 1 to 4,
    wherein the suspension containing the bioactive substance and the organism-derived material passes through an electric field-free region after passing through the first electric field region and before passing through the second electric field region.

6. The producing method according to claim 5,
    wherein a ratio T1/T0 of a first period T1 during which the suspension passes through the first electric field region to a third period T0 during which the suspension passes through the electric field-free region is 1/25,000 or more and less than 1.

7. The producing method according to any one of claims 1 to 6,
    wherein a suspension containing the bioactive substance and a suspension containing the organism-derived material before the introduction of the bioactive substance flow through flow channels different from each other, are mixed at a combining point of the respective flow channels, and then pass through the first electric field region and the second electric field region.

8. The producing method according to any one of claims 1 to 7,

    wherein the suspension passes through the first electric field region and the second electric field region by flowing inside the flow channel, and
    in a case where an area of a cross section of the flow channel orthogonal to a flow direction of the suspension is denoted by S [m$^2$], a circumference length of the cross section of the flow channel is denoted by C [m], and an average speed at which the suspension passes through the first electric field region and the second electric field region is denoted by u [m/s], a shear rate D [s$^{-1}$] defined by Expression (1) is 1 [s$^{-1}$] or more and 5,000 [s$^{-1}$] or less,

$$D = 2u \times C/S \cdots (1).$$

9. The producing method according to any one of claims 1 to 8, further comprising a step of causing the suspension to pass through at least one electric field region different from the first electric field region and the second electric field region.

10. The producing method according to any one of claims 1 to 9,

wherein the bioactive substance is DNA.

11. A producing method for a product, comprising:

a step of culturing the organism-derived material produced by the producing method according to any one of claims 1 to 10; and
a step of extracting a product that is produced by the organism-derived material.

12. The producing method according to claim 11,
wherein the product is a virus.

13. A voltage applying device comprising:

a flow channel for causing a liquid to flow;
a first pair of electrodes provided to face each other on wall surfaces of the flow channel; and
a second pair of electrodes provided to face each other on the wall surfaces of the flow channel, downstream of the first pair of electrodes in a flow direction of the liquid,
wherein a length of the first pair of electrodes in the flow direction is equal to or shorter than a length of the second pair of electrodes in the flow direction.

14. The voltage applying device according to claim 13,
wherein the ratio L1/L2 of the length L1 of the first pair of electrodes in the flow direction to the length L2 of the second pair of electrodes in the flow direction is 1/1,000 or more and 1 or less.

15. The voltage applying device according to claim 13 or 14,
wherein a ratio L1/L0 of the length L1 of the first pair of electrodes in the flow direction to a length L0 between the first pair of electrodes and the second pair of electrodes in the flow direction is 1/1,000 or more and less than 1.

16. The voltage applying device according to any one of claims 13 to 15,
wherein each of an inter-electrode distance of the first pair of electrodes and an inter-electrode distance of the second pair of electrodes is 10 $\mu$m or more and less than 10 mm.

17. The voltage applying device according to any one of claims 13 to 16,

wherein a first voltage is applied to the first pair of electrodes, and
a second voltage lower than the first voltage is applied to the second pair of electrodes.

18. The voltage applying device according to any one of claims 13 to 17, further comprising at least one pair of electrodes provided to face each other on the wall surfaces of the flow channel, which is different from the first pair of electrodes and the second pair of electrodes.

19. The voltage applying device according to any one of claims 13 to 18, wherein the flow channel has at least one combining point or branch point.

## FIG. 1A

## FIG. 1B

## FIG. 1C

## FIG. 2

## FIG. 3

# FIG. 4

## FIG. 5A

## FIG. 5B

FIG. 6

EP 3 985 099 A1

## FIG. 7A

ELECTRIC FIELD INTENSITY →

E1

E2

E3

E4

| ELECTRIC FIELD REGION 40A | ELECTRIC FIELD REGION 40B | ELECTRIC FIELD REGION 40C | ELECTRIC FIELD REGION 40D |

## FIG. 7B

ELECTRIC FIELD INTENSITY →

E1

E2

E3

E4

| ELECTRIC FIELD REGION 40A | ELECTRIC FIELD REGION 40B | ELECTRIC FIELD REGION 40C | ELECTRIC FIELD REGION 40D |

## FIG. 7C

## FIG. 7D

# FIG. 8

FIG. 9

# FIG. 10

## INTERNATIONAL SEARCH REPORT

| | International application No. |
|---|---|
| | PCT/JP2020/027109 |

### A. CLASSIFICATION OF SUBJECT MATTER
C12M 1/42(2006.01)i; C12P 1/00(2006.01)i; C12N 5/071(2010.01)i; C12N 15/87(2006.01)i
FI: C12N15/87 Z; C12M1/42; C12P1/00 Z; C12N5/071
According to International Patent Classification (IPC) or to both national classification and IPC

### B. FIELDS SEARCHED

Minimum documentation searched (classification system followed by classification symbols)
C12M1/42; C12P1/00; C12N5/071; C12N15/87

Documentation searched other than minimum documentation to the extent that such documents are included in the fields searched
|  |  |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)
JSTPlus/JMEDPlus/JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
|---|---|---|
| X | US 2017/0283761 A1 (CYTEQUEST, INC.) 05.10.2017 (2017-10-05) in particular, fig. 2, 4, 6, 7, paragraphs [0009]-[0011], [0029]-[0030], [0040]-[0042], [0074], example 1 | 13-16, 18, 19 |
| Y | in particular, fig. 2, 4, 6, 7, paragraphs [0009]-[0011], [0029]-[0030], [0040]-[0042], [0074], example 1 | 1-12, 17 |
| Y | JP 2015-008708 A (KYOTO UNIVERSITY) 19.01.2015 (2015-01-19) in particular, claims 1, 2, paragraphs [0026]-[0029] | 17 |
| A | in particular, claims 1, 2, paragraphs [0026]-[0029] | 1-16, 18, 19 |
| Y | WO 2016/017045 A1 (NEPA GENE CO., LTD.) 04.02.2016 (2016-02-04) in particular, example 2, table 6, fig. 6-10 | 1-12 |
| A | in particular, example 2, table 6, fig. 6-10 | 13-19 |

☒ Further documents are listed in the continuation of Box C.        ☒ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 27 August 2020 (27.08.2020) | 08 September 2020 (08.09.2020) |

| Name and mailing address of the ISA/ | Authorized officer |
|---|---|
| Japan Patent Office 3-4-3, Kasumigaseki, Chiyoda-ku, Tokyo 100-8915, Japan | |
| | Telephone No. |

Form PCT/ISA/210 (second sheet) (January 2015)

28

**INTERNATIONAL SEARCH REPORT**

| International application No. |
|---|
| PCT/JP2020/027109 |

C (Continuation).  DOCUMENTS CONSIDERED TO BE RELEVANT

| Category* | Citation of document, with indication, where appropriate, of the relevant passages | Relevant to claim No. |
|---|---|---|
| A | JP 2016-106526 A (INSTITUTE OF NATIONAL COLLEGES OF TECHNOLOGY JAPAN) 20.06.2016 (2016-06-20) entire text | 1-19 |
| A | JP 2019-097499 A (BEX CO., LTD.) 24.06.2019 (2019-06-24) entire text | 1-19 |
| A | JP 2011-528550 A (MAXCYTE, INC.) 24.11.2011 (2011-11-24) entire text | |
| A | WANG, H. et al., "Microfluidic Electroporation for Delivery of Small Molecules and Genes Into Cells Using a Common DC Power Supply", Biotechnology and Bioengineering, 2008, vol. 100, no. 3, pp. 579-586 entire text | 1-19 |

Form PCT/ISA/210 (continuation of second sheet) (January 2015)

**INTERNATIONAL SEARCH REPORT**

| International application No. |
| --- |
| PCT/JP2020/027109 |

**Box No. II     Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III     Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**     ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

INTERNATIONAL SEARCH REPORT
Information on patent family members

International application No.

PCT/JP2020/027109

| Patent Documents referred in the Report | Publication Date | Patent Family | Publication Date |
|---|---|---|---|
| US 2017/0283761 A1 | 05 Oct. 2017 | WO 2017/176764 A1 in particular, fig. 2, 4, 6, 7, paragraphs [0009]-[0011], [0029]-[0030], [0040]-[0042], [0074], example 1 EP 3440186 A1 | |
| JP 2015-008708 A | 19 Jan. 2015 | US 2015/0011008 A1 in particular, claims 1, 2, paragraphs [0098]-[0106] | |
| WO 2016/017045 A1 | 04 Feb. 2016 | US 2017/0080222 A1 in particular, example 2, table 6, fig. 6-10 EP 3176246 A1 CN 106459858 A | |
| JP 2106-106526 A | 20 Jun. 2016 | (Family: none) | |
| JP 2019-097499 A | 24 Jun. 2109 | (Family: none) | |
| JP 2011-528550 A | 24 Nov. 2011 | US 2012/0088842 A1 entire text WO 2010/009252 A1 EP 2310500 A1 CN 102124102 A | |

**INTERNATIONAL SEARCH REPORT**

| International application No. |
| --- |
| PCT/JP2020/027109 |

<Continuation of Box No. III>

Document 1: US 2017/0283761 A1 (CYTEQUEST, INC.) 05.10.2017 (2017-10-05) in particular, fig. 2, 4, 6, 7, paragraphs [0009]-[0011], [0029]-[0030], [0040]-[0042], [0074], example 1 & WO 2017/176764 A1 & EP 3440186 A1

Claims are classified into the two inventions below.

(Invention 1) Claims 1-12
Claims 1-13 have the special technical feature of a "method for producing an organism-derived object which is a human-derived cell introduced with bioactive substances, the method comprising a step in which a suspension containing bioactive substances and an organism-derived object passes through a first electric field area having a first electric field intensity and a step in which the suspension containing the same passes through a second electric field area having a second electric field intensity lower than the first electric field intensity," and thus are classified as invention 1.

(Invention 2) Claims 13-19
Claims 13-19 and claim 1 classified as invention 1 share the common technical feature of "making a liquid pass through a flow channel having a plurality of electric fields." However, since said technical feature does not make a contribution over the prior art in light of the disclosure of document 1 (in particular, see fig. 2, 4, 6, 7, paragraphs [0009]-[0011]), this technical feature cannot be considered a special technical feature.
Also, there are no other identical or corresponding special technical features between claims 13-19 and claim 1.
In addition, claims 13-19 are not dependent on claim 1, and are not substantially identical or equivalent to any of the claims classified as invention 1.
Therefore, claims 13-19 cannot be classified as invention 1.
In addition, claims 13-19 have the special technical feature of a "voltage application device provided with a flow channel for distributing a liquid, a first electrode pair, and a second electrode pair provided at a downstream side thereof, wherein the length of the first electrode pair in the distribution direction is equal to or shorter than that of the second electrode pair, and a voltage lower than that of the first electrode pair is applied to the second electrode pair," and thus are classified as invention 2.

Form PCT/ISA/210 (extra sheet) (January 2015)

**EP 3 985 099 A1**